# EUROPÄISCHE PATENTSCHRIFT

(11) **EP 1 641 477 B1**
(45) Veröffentlichungstag und Bekanntmachung des Hinweises auf die Patenterteilung: **15.04.2009**
(21) Anmeldenummer: 04739741.9
(22) Anmeldetag: 09.06.2004
(51) Int. Cl.: A61K 36/21, A61P 29/00

(54) **VERWENDUNG VON TEILEN ODER EINES EXTRAKTS VON AMARANTHUS BLITOIDES**
USE OF PARTS OR EXTRACT OF AMARATHUS BLITOIDES
UTILISATION DE PARTIES OU D'UN EXTRAIT D'AMARANTHUS BLITOIDES

(30) Priorität: 24.06.2003 DE 10329248; 10.07.2003 DE 10332281; 14.11.2003 US 519641 P
(43) Veröffentlichungstag der Anmeldung: 05.04.2006
(73) Patentinhaber: Nedwig, Eva-Maria, 10119 Berlin (DE); Milovanovic, Maria, 13507 Berlin (DE); Radoijcic, Jovan, 18412 Zitorada (YU)
(72) Erfinder: Nedwig, Eva-Maria, 10119 Berlin (DE); Milovanovic, Maria, 13507 Berlin (DE); Radoijcic, Jovan, 18412 Zitorada (YU)
(74) Vertreter: Ziebig, Marlene
(86) Internationale Anmeldenummer: PCT/EP2004/006235
(87) Internationale Veröffentlichungsnummer: WO 2004/112812

(56) Entgegenhaltungen:
- DE-A- 10 113 328
- PATENT ABSTRACTS OF JAPAN Bd. 1999, Nr. 03, 31. März 1999 (1999-03-31) & JP 10 330242 A (MIKIMOTO PHARMACEUT CO LTD), 15. Dezember 1998 (1998-12-15)
- PATENT ABSTRACTS OF JAPAN Bd. 2000, Nr. 08, 6. Oktober 2000 (2000-10-06) & JP 2000 143524 A (ICHIMARU PHARCOS CO LTD), 23. Mai 2000 (2000-05-23)

## Beschreibung

Die Erfindung betrifft die Verwendung von Teilen oder eines Extrakts von Amaranthus blitoides.

Amaranthus blitoides ist eine ursprünglich aus Süd- und Mittelamerika stammende Pflanze.

Amaranthus blitoides wurde und wird in Mittelamerika und in den Balkanländern in Form von Salaten und als Zusatz zu anderen Lebensmitteln sowie als Zusatz zu Tierfutter verwendet. Im Naturkosthandel finden sich Blätter und Samen dieser Pflanze in Müslis oder zu Grütze gekocht.

Weiterhin werden Amaranthaceen für die Herstellung von Lebensmittelfarbstoffen genutzt.

Eine Verwendung im pharmazeutischen Bereich ist aus der DE 101 13 328 A1 bekannt, in der Pflanzenkoagulate unter anderem auch von Amaranthaceen in Verbindung mit verschiedenen Pflanzenextrakten zur Behandlung von insbesondere Menstruationsstörungen eingesetzt werden.

Zur Behandlung entzündlicher Erkrankungen werden verschiedene Substanzen, wie zum Beispiel Lokalanästhetika, Glukokortikoide, Analgetika und Adstringentien verwendet. Diese Substanzen und ihre Zubereitungen werden entweder lokal, oral oder als Injektion verabreicht, wobei auch verschiedene Applikationsformen kombiniert werden. Zudem ist die Kombination mit antimikrobiell wirkenden Stoffen (Chemotherapeutika, Antibiotika) möglich.

Es ist Aufgabe der Erfindung, ein neues Präparat zur Behandlung von entzündlichen Erkrankungen bereitzustellen.

Diese Aufgabe wird durch die Merkmale des Anspruchs 1 gelöst.

Dazu ist erfindungsgemäß die Verwendung von Teilen oder einem Extrakt von Amaranthus blitoides zur Herstellung eines pharmazeutischen Präparates zur Behandlung von Hämorrhoiden, Herpes, Entzündungen der ableitenden Harnwege sowie Entzündungen der Prostata vorgesehen.

Es wurde nämlich überraschender Weise festgestellt, dass Teile oder Extrakte der Teile von Amaranthus blitoides in pharmokologischen Modellen starke analgetische, antiphlogistische und spasmolytische sowie auch laxierende Eigenschaften haben, wobei sich auch klinisch eine hohe Effektivität nachweisen lässt.

Amaranthus blitoides in getrockneter Form enthält neben verschiedenen Flavonoiden, Tannin sowie Vitamin C in einer Konzentration von 135 - 185 mg % und folgende Inhaltsstoffe:

| | |
|---|---|
| 41% | Stärke |
| 19% | Proteine |
| 18% | Fettöl |
| 2% | Zucker |
| 11% | Cellulose. |

Bei den erfindungsgemäß zu verwendenden Extrakten von Amaranthus blitoides handelt es sich vorzugsweise um wässrige Extrakte, da diese leicht - auch durch den Patienten selber in Form von Tee - herstellbar sind. Es können aber auch sonstige Extrakte wie beispielsweise alkoholische, alkoholisch-wässrige Extrakte oder Trockenextrakte verwendet werden.

Zur Herstellung von pharmazeutischen Präparaten bzw. Arzneimitteln können Teile von Amaranthus blitoides, flüssige oder zur Trockene eingeengte bzw. lyophilisierte Extrakte von Amaranthus blitoides zu Tee, Lösungen, Dragees, Tabletten oder Salben verarbeitet werden, die erfindungsgemäß neben den Bestandteilen des Amaranthus blitoides übliche Träger- und Hilfsstoffe enthalten können. Die erfindungsgemäßen Präparate werden dementsprechend oral, topisch, lokal oder parenteral angewendet.

Aufgrund der starken analgetischen, antiphlogistischen und spasmolytischen Eigenschaften sind die pharmazeutischen Präparate erfindungsgemäß zur Behandlung von entzündlichen Erkrankungen geeignet, nämlich Hämorrhoiden, Herpes, Entzündungen der ableitenden Harnwege sowie Entzündungen der Prostata.

Die Eignung der pharmazeutischen Präparate wurde im Rahmen von Untersuchungen festgestellt. Die Verwendung des pharmazeutischen Präparates für andere entzündliche Erkrankungen liegt für den Fachmann aufgrund der analgetischen, antiphlogistischen und spasmolytischen Eigenschaften nahe.

Weitere vorteilhafte Ausgestaltungen sind in den Unteransprüchen gekennzeichnet.

Nachstehend wird die Erfindung anhand von Beispielen näher erläutert.

### Beispiel 1

Zur Herstellung eines Tees bzw. eines wässrigen Extraktes von Amaranthus blitoides werden frische oder getrocknete Pflanzenteile mit kochendem Wasser übergossen. Den Aufguss lässt man für 5 - 10 min ziehen und seiht anschließend ab.

### Beispiel 2

Ein Trockenextrakt aus Amaranthus blitoides hat im Hot Plate Test nach oraler Gabe eine reaktionszeitverlängernde Wirkung. Diese ist Dosis- und zeitabhängig.

Die Wirkungsintensität ist nach Gabe von 33 und 100 mg/kg am höchsten. Amaranthus blitoides wirkt in diesem Dosisbereich mit einer 70%igen Hemmung der Reaktionszeit ebenso stark wie das methodenspezifische Referenzpharmakon Metamizol (s. Tab. 1). Diese Dosen entsprechen auch denen beim Menschen verwendeten.

**Tab. 1**

| Wirkung von Amaranthus blitoides 60 min. nach einmaliger oraler Gabe im Hot Plate Test an der Maus im Vergleich zur Kontrolle und Metamizol; es wurden 11 bis 12 Tiere pro Gruppe eingesetzt. | | | |
|---|---|---|---|
| Substanz | Dosis (mg/kg) | Reaktionszeit (sec)¹⁾ | Wirkung (%) |
| Kontrolle / 1 % Carboxy-methylcellulose | - | 6,07 ± 0,56 | - |
| Amaranthus blitoides | 33,00 | 10,39 ± 1,4^{*} | 71,17^{*} |
| | 100,00 | 10,73 ± 1,17^{*} | 76,77^{*} |
| | 330,00 | 9,71 ± 1,11^{*} | 59,97^{*} |
| Metamizol | 174,20 | 10,08 ± 1,19^{*} | 66,06^{*} |

| | | | |
|---|---|---|---|
| ¹⁾ Die Angabe erfolgt als Mittelwert ± Standardabweichung zum Mittelwert. ^{*)} p<0,05, MANN/WHITNEY/WILCOXON'S U-Test, zweiseitig | | | |

### Beispiel 3

Im Essigsäure-Writhing Test, einem anderen Modell zur Prüfung auf analgetische Wirkungen, zeigen Dosen von 33, 100, 330 mg/kg ebenfalls eine writhing-hemmende Wirkung. Die Wirkung ist ebenfalls dosis- und zeitabhängig. Auch in diesem Modell haben oral gegebene Extrakte aus Amaranthus blitoides die gleiche Wirkungsstärke wie Metamizol. Eine Dosis von 100 mg/kg Trockenextrakt aus Amaranthus blitoides ist der Dosis von 174,2 mg/kg Metamizol wirkungsäquivalent.

### Beispiel 4

Im Verhaltenstest an der Maus sind oral gegebene Extrakte aus Amaranthus blitoides zunächst unauffällig. In Dosen ab 100 mg/kg lassen sich leicht sedierende Wirkungen nachweisen. Die Wirkungen unterscheiden sich deutlich vom Chlorpromazin und Atropin. Die herausragende Wirkung in diesem Modell wird durch das Haffner-Phänomen gezeigt, das charakteristisch für analgetische Effekte ist.

Toxische Effekte wurden bis zu Dosen von 3300 mg/kg nach Amaranthus-Extrakt nicht beobachtet.

### Beispiel 5

Im Carrageenin induzierten Pfotenödem wurde eine geringe antiphlogistische Wirkung festgestellt. An der Darmmotilität der Maus ergaben sich keine Hinweise auf laxierende noch auf obstipierende Wirkungen. In vitro ließ sich am isolierten Meerschweinchenileum eine konzentrationsabhängige spasmolytische Wirkung nachweisen.

An der Ratte war die Defäkation erhöht, aber die Konsistenz der Fäces nicht beeinflusst.

### Beispiel 6

Die therapeutische Effizienz sowie die therapeutische Verträglichkeit wurden an Patienten mit äußeren und inneren Hämorrhoiden überprüft.

Dabei wurden 48 Patienten mit der Standardtherapie behandelt und 48 Patienten erhielten zusätzlich alle 4 Stunden eine Extraktmenge, die etwa 5 g Trockendroge von Amaranthus blitoides entsprach. Dieses Regime erfolgte am ersten, zweiten, dritten, siebenten und vierzehnten Tag der Behandlung. Die Bewertung des Präparates erfolgte auf der Basis von subjektiven Einschätzungen der Patienten und des Arztes (Wirksamkeitsscores) und durch objektive Kriterien (photographische Dokumentation).

Die Behandlungsergebnisse sind in Tabelle 2 wiedergegeben. Es ist ersichtlich, dass bei etwa 85 % der Patienten die Ergebnisse gut, sehr gut bzw. ausgezeichnet waren. Diese Patienten geben bereits kurze Zeit nach der ersten Einnahme des Extrakts deutliches Nachlassen der Schmerzen und weiterer Beschwerden an.

Nur bei einem Patienten trat eine Verschlechterung des Zustandes ein. Als Nebenwirkung wurde in wenigen Fällen Reizungen des Magens, Übelkeit und in zwei Fällen Diarrhoe angegeben. Insgesamt wurde das Präparat gut vertragen.

Die Therapie kann ergänzt werden durch die topische Anwendung einer Salbe, die Trockenextrakt aus Amaranthus blitoides enthält.

**Tab. 2 Behandlungsergebnisse durch Extrakte von Amaranthus blitoides bei Patienten mit Hämorrhoiden, Stadium 1 und 2**

| Behandlungsergebnisse | Zahl der Patienten | Prozent |
|---|---|---|
| Verschlechterung (ungenügend) | 1 | 2,07 |
| keine Veränderung (unbefriedigend) | 4 | 8,28 |
| Besserung (gut) | 10 | 20,71 |
| Bedeutende Besserung (sehr gut) | 22 | 45,75 |
| Verschwinden der Beschwerden (ausgezeichnet) | 11 | 22,91 |
| Gesamt | 48 | 99,72 |

### Beispiel 7

Extrakte von Amaranthus blitoides wurden an 100 Patienten mit einer akuten oder chronischen Infektion der Harnwege (Pyelonephritis, Zystitis, Steinbildung) oder der Prostata untersucht. Vor Beginn der Therapie wurden alle Patienten kompletten Laboranalysen des Blutes und Urins unterzogen, bei Prostataerkrankungen wurde eine Spermakultur angelegt.

Die Infektion war in den meisten Fällen durch E. coli und Proteus mirabilis verursacht worden, während in 10 Fällen Pseudomonas isoliert wurde. Staphylococcus aureus war bei den erhaltenen Spermakulturen dominant.

Bei allen Patienten wurde eine Behandlung durchgeführt, bei der alle 6 Stunden ein Extrakt verabreicht wurde, der 5 g Trockendroge von Amaranthus blitoides entsprach.

Dies wurde mit sechs Einzelgaben wiederholt.

Bei 42 von 65 Patienten mit Harnwegsinfektionen erwiesen sich die Kulturen als steril, oder die Anzahl der Mikro-organismen war signifikant verringert im Vergleich zum Zustand vor der Therapie. Bei 10 Patienten mit Prostataerkrankungen besserten sich die Spermakulturen ebenfalls.

Bei 5 von 10 Patienten mit nachgewiesenen Pseudomonas-Infektionen war der Zustand signifikant verbessert, während bei den restlichen 5 Patienten die Befunde gleich geblieben waren, was eine Fortsetzung der Antibiotika-Therapie erforderte.

Subjektive Beschwerden waren bei 17 von 25 Patienten signifikant verringert. Es wurden keine Nebenwirkungen verzeichnet und keine Veränderungen der biochemischen Blut-parameter(Harnstoff, Kreatinin, Glucose, Hepatogramm, Phosphatase, Cholesterol, Triglyceride, Blutbild, Blutsenkung).

### Beispiel 8

Auch bei Herpes simplex wurde eine Salbe, die den Extrakt von Amaranthus blitoides enthielt, mit Erfolg angewendet.

## Patentansprüche

1. Verwendung von Teilen oder eines Extrakts von Amaranthus blitoides zur Herstellung eines pharmazeutischen Präparates zur Behandlung von Hämorrhoiden, Herpes, Entzündungen der ableitenden Harnwege sowie Entzündungen der Prostata.

2. Verwendung nach Anspruch 1,
**dadurch gekennzeichnet, dass**
ein wässriger Extrakt verwendet wird.

3. Verwendung nach Anspruch 1,
**dadurch gekennzeichnet, dass**
ein alkoholischer oder alkoholisch-wässriger Extrakt verwendet wird.

4. Verwendung nach Anspruch 1,
**dadurch gekennzeichnet, dass**
ein Trockenextrakt verwendet wird.

5. Verwendung nach einem der Ansprüche 1 bis 3,
**dadurch gekennzeichnet, dass**
ein zur Trockene eingeengter bzw. lyophilisierter Extrakt verwendet wird.

6. Verwendung nach einem der Ansprüche 1 bis 5,
**dadurch gekennzeichnet, dass**
die Verwendung in Form eines Tees oder eines Instant-Tees erfolgt.

## Claims

1. Use of parts or of an extract of Amaranthus blitoides in the production of a pharmaceutical preparation for the treatment of hemorrhoids, herpes, inflammations of the efferent urinary tract as well as inflammations of the prostate.

2. The use according to claim 1,
**characterized in that**
an aqueous extract is used.

3. The use according to claim 1,
**characterized in that**
an alcoholic or alcoholic-aqueous extract is used.

4. The use according to claim 1,
**characterized in that**
a dry extract is used.

5. The use according to any of claims 1 to 3,
**characterized in that**
an extract concentrated to dryness or a lyophilized extract is used.

6. The use according to any of claims 1 to 5,
**characterized in that**
the use is in the form of a tea or an instant tea.

## Revendications

1. Utilisation de parties ou d'un extrait d'Amaranthus blitoides pour préparer une préparation pharmaceutique destinée au traitement des hémorroïdes, de l'herpès, des inflammations des voies urinaires déférentes ainsi que des inflammations de la prostate.

2. Utilisation selon la revendication 1
**caractérisée en ce**
**qu'**on utilise un extrait aqueux.

3. Utilisation selon la revendication 1
**caractérisée en ce**
**qu'**on utilise un extrait alcoolique ou un extrait alcoolique-aqueux.

4. Utilisation selon la revendication 1
**caractérisée en ce**
**qu'**on utilise un extrait sec.

5. Utilisation selon l'une quelconque des revendications 1 à 3,
**caractérisée en ce**
**qu'**on utilise un extrait évaporé ou lyophilisé à sec.

6. Utilisation selon l'une quelconque des revendications 1 à 5,
**caractérisée en ce que**
l'utilisation s'effectue sous forme d'une tisane ou d'une tisane instantanée.
